(19) Europäisches Patentamt
European Patent Office
Office européen des brevets

(11) Veröffentlichungsnummer: **0 187 284**
**A2**

(12) # EUROPÄISCHE PATENTANMELDUNG

(21) Anmeldenummer: 85115634.9

(22) Anmeldetag: 09.12.85

(51) Int. Cl.⁴: **C 07 D 213/61**
C 07 D 213/82

(30) Priorität: 20.12.84 DE 3446553
13.06.85 DE 3521128
24.10.85 DE 3537762

(43) Veröffentlichungstag der Anmeldung: 16.07.86 Patentblatt 86/29

(84) Benannte Vertragsstaaten: BE CH DE FR GB IT LI NL

(71) Anmelder: BAYER AG
Konzernverwaltung RP Patentabteilung
D-5090 Leverkusen 1 Bayerwerk(DE)

(72) Erfinder: Marzolph, Gerhard, Dr.
Semmelweisstrasse 87a
D-5000 Köln 80(DE)

(72) Erfinder: Streicher, Willi, Dr.
Andreas-Gryphius-Strasse 7
D-5000 Köln 80(DE)

(72) Erfinder: Blank, Heinz Ulrich, Dr.
Am Geusfelde 35
D-5068 Odenthal(DE)

(54) **Verfahren zur Herstellung von Pyridin-carbonsäure-N-tert.-alkylamiden.**

(57) Pyridin-carbonsäure-N-tert.-alkylamide können durch Umsetzung eines Pyridincarbonsäurenitrils mit einem tertiäre Carbeniumionen liefernden Alkylierungsmittel in Gegenwart von 50 - 94 %iger Schwefelsäure bei 0 bis 100°C hergestellt werden. Bevorzugt wird ohne weiteres Lösungsmittel gearbeitet.

EP 0 187 284 A2

0187284

BAYER AKTIENGESELLSCHAFT 5090 Leverkusen, Bayerwerk

Konzernverwaltung RP Ha/by-c
Patentabteilung

## Verfahren zur Herstellung von Pyridin-carbonsäure-N-tert.-alkylamiden

Die vorliegende Erfindung betrifft ein Verfahren zur Herstellung von Pyridin-carbonsäure-N-tert.-alkylamiden durch Umsetzung von Cyanopyridinen mit tert.-Alkoholen in Gegenwart von Schwefelsäure.

N-tert.-Alkylamide von aliphatischen und aromatischen Carbonsäuren sind durch Umsetzung der entsprechenden aliphatischen oder aromatischen Nitrile mit tertiären Alkoholen in der als "Ritter-Reaktion" bekannten Umsetzung gut zugänglich (Organic Reactions 17, 213 ff. (1969)).

Die Umsetzung von Cyanopyridinen in dieser Reaktion ist durch die Salzbildung am Pyridin-N-Atom komplizierter als bei anderen Substraten. Die hierbei auftretende Neutralisationswärme erschwert die Beherrschung des Reaktionsablaufs.

Überhitzte Stellen im Reaktionsansatz können sodann zur Verseifung der Nitrilgruppe führen; weiterhin ist die Abspaltung des tert.-Alkylrestes vom N-Atom in Form des zugehörigen Olefins zu befürchten.

Die Ritter-Reaktion an Cyanopyridinen ist daher nur unter Anlegung spezieller Reaktionsbedingungen untersucht.

In J. Chem. Soc. (C) 1967, 1558 ist die Reaktion von 3-Cyanopyridin mit mehr als 10facher molarer Menge an tert.-Butylacetat unter Zusatz von Perchlorsäure in 18 Stunden bei 20°C beschrieben. Diese Reaktion erfordert also lange Reaktionszeiten, sehr große Überschüsse der teuren Reaktionskomponente tert.-Butylacetat, die zu tert.-Butanol und Essigsäure verseift wird, und wird unter Einsatz der technisch unerwünschten Perchlorsäure durchgeführt.

In J. Am. Chem. Soc. 1952, 763 wird 3-Cyanopyridin mit $\alpha,\alpha$-Dimethyl-ß-phenethyl-alkohol in Gegenwart von konzentrierter Schwefelsäure und Zusatz von Eisessig als Lösungsmittel umgesetzt. Die Ausbeute beträgt 71 %. Die genannte Arbeitsweise hat den Nachteil, daß das Nitril nur in 18 %iger Lösung umgesetzt wird, wodurch die Raum-Zeit-Ausbeute negativ beeinflußt wird. Außerdem gelangt die Essigsäure bei der Aufarbeitung ins Abwasser und führt bei der Neutralisation der Mischung zu einem unerwünscht hohen Verbrauch an Base.

Es besteht jedoch ein Bedarf für ein ökonomisches Verfahren zur Herstellung von Pyridin-carbonsäure-N-tert.-alkylamiden, das bei geringem Chemikalienverbrauch und niedriger Abwasserbelastung die gewünschten Amide in hoher Ausbeute liefert und die genannten Komplikationen der Reaktion vermeidet.

Es wurde nun ein Verfahren zur Herstellung von Pyridin-N-tert.-alkylamiden der Formel

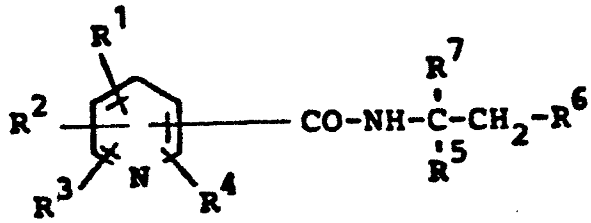

(I),

in der

$R^1$, $R^2$, $R^3$ und $R^4$ unabhängig voneinander Wasserstoff, Halogen, Alkyl, Alkoxy, Phenyl, Benzyl oder Nitro bedeuten und zwei der Reste $R^1$ bis $R^4$ gemeinsam einen annellierten Benzolring bedeuten können und

$R^5$, $R^6$ und $R^7$ unabhängig voneinander geradkettige oder verzweigte Alkylgruppen mit 1-8 C-Atomen bedeuten, wobei weiterhin $R^5$ und $R^6$ gemeinsam mit den C-Atomen, die sie substituieren, einen aliphatischen Ring von 5-8 Ringgliedern bilden können, $R^7$ weiterhin ein gegebenenfalls substituierter Phenylrest sein kann und $R^6$ außerdem Wasserstoff sein kann,

durch Umsetzung eines Pyridincarbonsäurenitrils der Formel

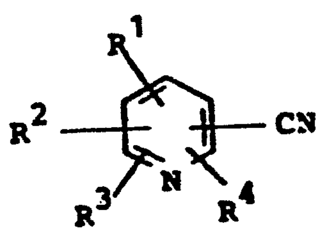

(II),

in der

$R^1$ bis $R^4$ die angegebene Bedeutung besitzen,

mit einem tertiäre Carbeniumionen liefernden Alkylierungsmittel der Formel

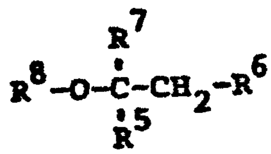

(III)

oder der Formel

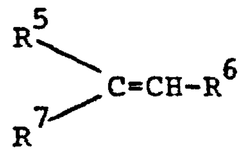

(IV),

in denen

$R^5$ bis $R^7$ die angegebene Bedeutung besitzen und

$R^8$ Wasserstoff, Alkyl oder Acyl bedeutet,

in Gegenwart von Schwefelsäure gefunden, das dadurch gekennzeichnet ist, daß die Reaktion bei 0 - 100°C und einer Konzentration der Schwefelsäure von 50 - 94 Gew.-% durchgeführt wird.

Zur Durchführung des erfindungsgemäßen Verfahrens wird 50-94 gew.-%ige Schwefelsäure, bevorzugt 60-90 gew.-%ige Schwefelsäure eingesetzt.

Als Menge der Schwefelsäure sei beispielsweise 0,5-3,0, bevorzugt 0,8-2,7 Mol, besonders bevorzugt 1,0-2,5 Mol pro Mol des Nitrils genannt. Selbstverständlich kann das Verfahren auch mit Schwefelsäuremengen außerhalb der genannten Bereiche durchgeführt werden; hierbei werden gegebenenfalls schlechtere Ergebnisse erzielt.

Als Halogen sei beispielsweise Fluor, Chlor, Brom oder Jod, bevorzugt Fluor, Chlor oder Brom, genannt.

Als Alkyl seien beispielsweise geradkettige oder verzweigte gesättigte Kohlenwasserstoffreste mit 1-8, bevorzugt 1-4, besonders bevorzugt 1-2 C-Atomen genannt, wie Methyl, Ethyl, Propyl, Isopropyl, Butyl, Isobutyl, Hexyl, Octyl. Alkyl kann ein- oder mehrfach mit Fluor oder Chlor substituiert sein, beispielsweise als Fluormethyl, Trifluormethyl, Chlormethyl, Dichlormethyl, Trichlormethyl, Perfluorethyl, Perchlorethyl und ihre Homologen.

Als Alkoxy seien Reste von Alkanolen mit 1-8, bevorzugt 1-4, besonders bevorzugt 1-2 C-Atomen. Die im Alkoxy vorkommenden Alkylgruppen sind die gleichen, wie oben genannt.

Wenn zwei der Reste $R^1$ bis $R^4$ einen annellierten Benzolring bedeuten, gelangt man in die Reihe der Chinolin- oder Isochinolin-Verbindungen.

Die Reste $R^5$ und $R^6$ können mit den durch sie substituierten C-Atomen einen aliphatischen Ring mit 5-8, bevorzugt 5-6 Ringgliedern bilden.

Als Acyl sei solches mit 2-7, bevorzugt 2-4 C-Atomen, in besonders bevorzugter Weise Acetyl genannt.

Als Pyridin-carbonsäurenitrile (Cyano-pyridine) seien bevorzugt solche der Formel

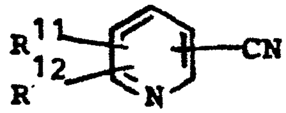

(V)

genannt,

in der

$R^{11}$ und $R^{12}$ unabhängig voneinander Wasserstoff, Halogen, Alkyl, Alkoxy oder Nitro bedeuten.

Als Cyano-pyridine seien besonders bevorzugt solche der Formel

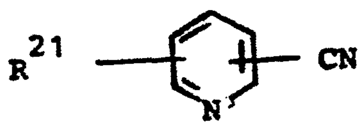

(VI)

genannt,

in der

$R^{21}$ Wasserstoff, Halogen, Alkyl, Alkoxy oder Nitro bedeutet.

In ganz besonders bevorzugter Weise bedeutet $R^{21}$ Wasserstoff, Fluor, Chlor, Brom, Methyl, Ethyl, Methoxy, Ethoxy oder Nitro.

Als Alkylierungsmittel der Formeln (III) und (IV) können tertiäre Alkanole, Ether oder Ester solcher tertiären Alkanole oder an der Doppelbindung verzweigte iso-Alkene genannt werden. Allen genannten Verbindungsklassen gemeinsam ist ihre Fähigkeit zur Bildung von Carbeniumkationen der Formel

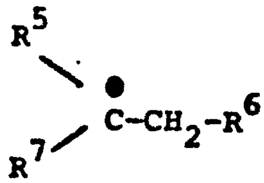

(VII)

mit den angegebenen Bedeutungen für $R^5$ bis $R^7$.

Als bevorzugte Alkylierungsmittel seien solche der Formeln

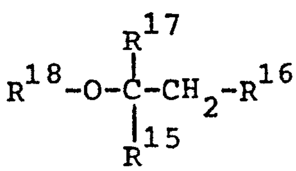

oder

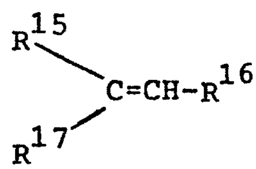

(VIII) (IX)

genannt,

in denen

$R^{15}$ und $R^{17}$ unabhängig voneinander geradkettiges $C_1$-$C_4$-Alkyl bedeuten,

$R^{16}$ für Wasserstoff oder geradkettiges oder verzweigtes $C_1$-$C_4$-Alkyl steht und

$R^{18}$ Wasserstoff, $C_1$-$C_4$-Alkyl oder Acetyl bedeutet,

wobei weiterhin $R^{15}$ und $R^{16}$ gemeinsam mit den C-Atomen, die sie substituieren, einen aliphatischen Ring von 5-6 Ringgliedern bilden können.

In ganz besonders bevorzugter Weise bedeuten $R^{15}$, $R^{16}$ und $R^{17}$ unabhängig voneinander Methyl oder Ethyl, $R^{16}$ kann zusätzlich Wasserstoff bedeuten, und $R^{18}$ bedeutet Wasserstoff, Methyl, Ethyl oder Acetyl.

Als Alkylierungsmittel seien beispielsweise tert.-Butanol, tert.-Amylalkohol, Methyl-tert.-butylether, Methyl-tert.-amylether, tert.-Butylacetat, tert.-Amylacetat, Isobuten, tert.-Amylen, 1-Methylcyclopent-1-en, 1-Methylcyclohex-1-en, 1-Methylcyclopentanol-1 oder 1-Methyl-cyclohexanol-1 genannt.

Das Mengenverhältnis des Alkylierungsmittels zum Nitril kann weitgehend beliebig gewählt werden, jedoch ist es in den meisten Fällen vorteilhaft, nicht weniger als etwa 0,8 und nicht mehr als 4 Mol Alkylierungsmittel je Mol Nitril zu nehmen. Vorzugsweise werden 0,8-2,5 Mol, insbesondere 1,0-2,0 Mol je Mol des Nitrils verwendet.

Das erfindungsgemäße Verfahren wird bei einer Temperatur von 0-100°C, bevorzugt von 10-95°C, besonders bevorzugt von 20-90°C, durchgeführt. Im Rahmen des genannten Temperaturbereichs kann die Reaktionstemperatur während der Reaktion erhöht oder erniedrigt werden.

Das erfindungsgemäße Verfahren kann grundsätzlich in einem bisher für die Ritter-Reaktion benutzten organischen Lösungsmittel durchgeführt werden, z.B. in Eisessig. Überraschenderweise kann jedoch erfindungsgemäß die Lösungsmittelmenge gegenüber herkömmlichen

Ansätzen der Ritter-Reaktion bedeutend verringert werden. In bevorzugter Weise wird ohne weiteres Lösungsmittel gearbeitet.

Für die Durchführung des erfindungsgemäßen Verfahrens sind verschiedene Varianten möglich.

Das erfindungsgemäße Verfahren kann beispielsweise so durchgeführt werden, daß das Cyanopyridin, das Alkylierungsmittel und die Schwefelsäure simultan bei der Reaktionstemperatur zudosiert werden.

In einer weiteren Variante kann auch ein Gemisch aus Cyanopyridin und dem Alkylierungsmittel vorgelegt werden und dazu bei Reaktionstemperatur die Schwefelsäure dosiert werden. Insbesondere, wenn im unteren Teil der genannten Bereiche für die Temperatur und/oder für die $H_2SO_4$-Menge gearbeitet werden soll, werden nach dieser Variante gute Ergebnisse erzielt.

In noch einer weiteren Variante wird die Schwefelsäure vorgelegt, und bei Reaktionstemperatur werden das Cyanopyridin und das Alkylierungsmittel simultan zudosiert.

Schließlich können auch die Schwefelsäure und das Alkylierungsmittel vorgelegt und das Cyanopyridin zudosiert oder das Cyanopyridin vorgelegt und ein Gemisch aus Schwefelsäure und Alkylierungsmittel zudosiert werden. Diese Variante gibt besonders dann gute Ergebnisse, wenn im unteren Teil des genannten Bereiches der $H_2SO_4$-Konzentration gearbeitet wird.

Weiterhin kann ein Gemisch aus Cyanopyridin und Schwefelsäure vorgelegt und das Alkylierungsmittel zudosiert oder das Alkylierungsmittel vorgelegt und das Gemisch aus Cyanopyridin und Schwefelsäure zudosiert werden. Diese Variante wird beispielsweise bei 20 - 90°C, bevorzugt bei erhöhter Temperatur, beispielsweise bei 50 - 90°C, durchgeführt. Aber auch in der Nähe der Raumtemperatur und einer $H_2SO_4$-Menge von etwa 1,8 Mol/Mol Cyanopyridin oder mehr werden gute Ergebnisse erzielt.

Die Vorlage von Cyanopyridin und Schwefelsäure und das anschließende Zutropfen des Alkylierungsmittels ist eine bevorzugte Durchführungsvariante.

Man erzielt mit Hilfe des erfindungsgemäßen Verfahrens hohe Ausbeuten bei einem minimalen Verbrauch an Reaktionskomponenten und kann die Reaktion überraschenderweise ohne weitere Hilfslösemittel durchführen, so daß deren Entsorgung das Verfahren nicht belastet.

Das erfindungsgemäße Verfahren kann grundsätzlich diskontinuierlich, beispielsweise in einem Autoklaven, Druckkessel oder Rührkessel, oder auch kontinuierlich, beispielsweise in einem beheizbaren oder kühlbaren und druckfesten Strömungsrohr durchgeführt werden. Ein erhöhter Druck als der Normaldruck braucht nur angelegt zu werden, wenn bei erhöhter Temperatur niedrig siedende Reaktionspartner in der flüssigen Phase gehalten werden sollen.

Nach Beendigung der Reaktion wird das Reaktionsgemisch neutralisiert. Zur Neutralisation können als Basen beispielsweise die Hydroxide oder Carbonate der Metalle der ersten Hauptgruppe des Periodensystems (Mendelejew) eingesetzt werden. Bei flüssigen Produkten kann nach der Neutralisation die organische Phase von der wäßrigen Phase abgetrennt werden; bei festen Reaktionsprodukten werden diese abgesaugt oder abgepreßt oder durch geeignete Extraktionsmittel aus dem Reaktionsgemisch isoliert und nach dem Entfernen des Extraktionsmittels beispielsweise durch Destillation gereinigt. Das so gewonnene Produkt fällt in hoher Reinheit und in sehr guten Ausbeuten an.

Pyridin-carbonsäure-N-tert.-alkylamide werden in neuen herbiziden Wirkstoffkombinationen gemeinsam mit Fotosynthesehemmer-Herbiziden eingesetzt (DE 3 332 272).

Weiterhin können durch eine dem Fachmann bekannte hydrolytische Spaltung gleichzeitig tert.-Alkylamine ("Ritter-Amine") und gegebenenfalls substituierte Pyridincarbonsäuren erhalten werden. Pyridincarbonsäuren werden beispielsweise zur Synthese von Malariamitteln vom Typ des Isonicotinsäure-hydrazids (Isoniazid) verwendet.

Beispiele 1-9

Allgemeine Arbeitsvorschrift zur Tabelle 1

2-Cyanopyridin und tert.-Butanol bzw. tert.-Butylmethylether werden bei 50°C zusammen vorgelegt. Unter Rühren tropft man bei dieser Temperatur innerhalb von 40 min die Schwefelsäure zu. Man hält durch Heizen die Innentemperatur auf 50°C und läßt 5 h nachrühren. Nach Reaktionsende wird auf Raumtemperatur abgekühlt. Das Reaktionsgemisch wird mit Natronlauge neutralisiert und mit Methylenchlorid extrahiert. Die organische Phase wird über Natriumsulfat getrocknet. Danach wird das Lösungsmittel abgezogen und der Rückstand gaschromatographisch analysiert.

Tabelle 1 Umsetzungen von 2-Cyanopyridin mit tert.-Butanol bzw. tert.-Butylmethylether in Gegenwart von Schwefelsäure bei 50°C nach 5 h Reaktionszeit

| Bsp. Nr. | Konzentration der Schwefelsäure (%) | Schwefelsäure (Mol) | 2-Cyanopyridin (Mol) | tert.-Butanol (Mol) | tert.-Butylmethylether (Mol) | Gewicht des Rückstandes (g) | Gehalt (%) * | Ausbeute (% d.Th.) |
|---|---|---|---|---|---|---|---|---|
| 1 | 83,9 | 0,4 | 0,2 | | 0,3 | 65,1 | 89,1 | 81,4 |
| 2 | 94,0 | 0,4 | 0,2 | | 0,4 | 65,8 | 78,4 | 72,5 |
| 3 | 85,0 | 0,4 | 0,2 | | 0,4 | 65,1 | 96,0 | 87,7 |
| 4 | 85,0 | 0,4 | 0,2 | 0,3 | | 65,9 | 91,0 | 84,1 |
| 5 | 92,0 | 0,4 | 0,2 | 0,3 | | 66,6 | 76,0 | 71,0 |
| 6 | 92,0 | 0,3 | 0,2 | 0,3 | | 66,6 | 75,0 | 70,1 |
| 7 | 83,9 | 0,34 | 0,2 | 0,3 | | 66,6 | 91,2 | 85,3 |
| 8 | 83,9 | 0,36 | 0,2 | 0,3 | | 65,1 | 95,2 | 87,0 |
| 9 | 83,9 | 0,32 | 0,2 | 0,3 | | 68,6 | 86,6 | 83,4 |

* nach gaschromatografischer Analyse

Beispiel 10

104,0 g (1,0 Mol) 2-Cyanopyridin und 111,0 g (1,5 Mol) tert. Butanol werden bei 50°C zusammen vorgelegt. Unter Rühren tropft man bei dieser Temperatur innerhalb von 40 min 210,3 g (1,8 Mol) 83,9 %ige Schwefelsäure zu. Man hält durch Heizen die Innentemperatur auf 50°C und läßt 5 h nachrühren. Danach wird auf Raumtemperatur abgekühlt und das Reaktionsgemisch mit 380 ml Wasser verdünnt. Anschließend wird das Gemisch mit 337,0 g 45 %iger Natronlauge neutralisiert. Das Produkt, das sich als Öl oben abscheidet, wird bei 40°C abgetrennt. Zur weiteren Reinigung wird das Rohprodukt über eine 10 cm -Vigreux-Kolonne unter Vakuum destilliert. Bei einer Kopftemperatur von 82°C bis 84°C und einem Druck von 1,1 mbar gehen 160,9 g 2-Picolinsäure-N-tert.-butylamid über. Der Gehalt des Destillats beträgt 94,7 %.

Ausbeute: 85,6 % der theoretischen Ausbeute

Beispiel 11

Analog Beispiel 10 werden 104,0 g (1,0 Mol) 2-Cyanopyridin und 176,0 g (2,0 Mol) tert.-Butylmethylether mit 230,6 g 85 %iger (2,0 Mol) Schwefelsäure umgesetzt. Nach Destillation erhält man 159,3 g 2-Picolinsäure-N-tert.-butylamid mit einem Gehalt von 96,2 %, entsprechend einer Ausbeute von 86,1 % d. Th.

Beispiele 12-18

Allgemeine Arbeitvorschrift zur Tabelle 2

In die vorgelegte Schwefelsäure wird unter Eiskühlung das 2-Cyanopyridin so zudosiert, daß die Reaktionstemperatur erreicht wird. Unter Rühren tropft man bei dieser Temperatur innerhalb von 75 min tert.-Butanol zu. Man hält durch Heizen die Innentemperatur auf Reaktionstemperatur und rührt bis zum Reaktionsende weiter. Danach wird auf Raumtemperatur abgekühlt und das Reaktionsgemisch mit Natronlauge neutralisiert. Anschließend wird mit Methylenchlorid extrahiert und die organische Phase über Natriumsulfat getrocknet. Nach Entfernen des Lösungsmittels wird der Rückstand gaschromatographisch analysiert.

Tabelle 2 Umsetzung von 2-Cyanopyridin mit tert.-Butanol in Gegenwart von Schwefelsäure

| Bsp. Nr. | Temperatur °C | Reaktionszeit (h) | Konzentration d. Schwefelsäure (%) | Schwefelsäure (Mol) | 2-Cyanopyridin (Mol) | tert.-Butanol (Mol) | Gewicht d. Rückstandes (g) | Gehalt (%) | Ausbeute (% d.Th.) |
|---|---|---|---|---|---|---|---|---|---|
| 12 | 50 | 0,3 | 93,5 | 0,4 | 0,2 | 0,3 | 34,0 | 95,4 | 91 |
| 13 | 40 | 1 | 90,5 | 0,4 | 0,2 | 0,3 | 33,4 | 95,4 | 90,5 |
| 14 | 60 | 5 | 89,3 | 0,3 | 0,2 | 0,22 | 34,0 | 96,5 | 92,0 |
| 15 | 50 | 6 | 90,5 | 0,3 | 0,2 | 0,22 | 33,8 | 94,7 | 90 |
| 16 | 50 | 6 | 89,3 | 0,3 | 0,2 | 0,22 | 34,0 | 97,6 | 93,0 |
| 17 | 50 | 6 | 83,9 | 0,3 | 0,2 | 0,22 | 34,7 | 74,4 | 72,4 |
| 18 | 50 | 6 | 83,9 | 0,36 | 0,2 | 0,30 | 33,8 | 97,5 | 92,5 |

Beispiel 19

493,8 g 89,3 %ige Schwefelsäure (4,5 Mol) werden bei Raumtemperatur vorgelegt. Unter Eiskühlung werden 312,3 g (3,0 Mol) 2-Cyanopyridin so zudosiert, daß eine Innentemperatur von 50°C erreicht wird. Unter Rühren tropft man bei dieser Temperatur innerhalb von 75 min 244,2 g (3,3 Mol) tert.-Butanol zu. Man hält durch Heizen die Innentemperatur auf 50°C und läßt 6 h weiterrühren. Nach Reaktionsende wird auf Raumtemperatur abgekühlt und das Reaktionsgemisch mit 950 ml Wasser verdünnt. Anschließend wird mit 842,0 g 45 %iger Natronlauge auf pH 7 gestellt. Das Produkt, das sich als Öl oben abscheidet, wird bei 40°C abgetrennt. Zur weiteren Reinigung wird das Rohprodukt über eine 10 cm-Vigreux-Kolonne unter Hochvakuum destilliert. Nach Destillation erhält man 502,4 g 2-Picolinsäure-N-tert.-butylamid mit einem Gehalt von 98,4 %.

Ausbeute: 92,4 % der theoretischen Ausbeute

Beispiel 20

493,8 g 89,3 %ige Schwefelsäure (4,50 Mol) werden bei Raumtemperatur vorgelegt. Unter Eiskühlung werden 312,3 g (3,0 Mol) 4-Cyanopyridin so zudosiert, daß eine Innentemperatur von 50°C erreicht wird. Unter Rühren tropft man bei dieser Temperatur innerhalb von 75 min 244,2 g (3,0 Mol) tert.-Butanol zu. Man hält durch Heizen die

Innentemperatur auf 50°C und läßt 6 h weiterrühren. Nach Reaktionsende wird auf Raumtemperatur abgekühlt und das Reaktionsgemisch mit 950 ml Wasser verdünnt. Anschließend wird mit 842,0 g 45 %iger Natronlauge auf pH 7 gestellt. Das ausgefallene Produkt wird abgesaugt, mit Wasser gewaschen und bei 50°C im Vakuumtrockenschrank getrocknet. Man erhält 514,1 g Isonicotinsäure-N-tert.-butylamid mit einem Gehalt von 96,8 %.

Ausbeute: 93,2 % der theoretischen Ausbeute

Fp.: 120 bis 122,5°C

Beispiel 21

Analog Beispiel 20 wurden 312,3 g (3,0 Mol) 3-Cyanopyridin mit 244,2 g (3,3 Mol) tert.-Butanol in 493,8 g 89,3 %iger Schwefelsäure (4,5 Mol) umgesetzt. Man erhält 507,2 g Nicotinsäure-N-tert.-butylamid mit einem Gehalt von 96,1 %.

Ausbeute: 91,3 % der theoretischen Ausbeute

Fp.: 82°C.

Beispiel 22

Analog Beispiel 21 wurden 415,5 g (3,0 Mol) 3-Chlor-2-cyanopyridin mit 244,2 g (3,3 Mol) tert.-Butanol umgesetzt. Man erhält 601,6 g 3-Chlor-picolinsäure-N-tert.-butylamid mit einem Gehalt von 95,8 %.

Ausbeute: 90,4 % der theoretischen Ausbeute

Fp.: 103 bis 106°C

Beispiel 23

421,0 g 83,9 %ige Schwefelsäure (3,6 Mol) werden bei 50°C vorgelegt. Unter Eiskühlung werden bei dieser Temperatur innerhalb von 75 min simultan 208,0 g (2,0 Mol) 2-Cyanopyridin und 222,0 g (3,0 Mol) tert.-Butanol zugegeben. Man hält durch Heizen die Innentemperatur auf 50°C und läßt 7 h weiterrühren. Nach Reaktionsende wird auf Raumtemperatur abgekühlt und das Reaktionsgemisch mit 760 ml Wasser verdünnt. Anschließend wird mit 673,0 g 45 %iger Natronlauge auf pH 7 gestellt. Das Produkt, das sich als Öl oben abscheidet, wird bei 40°C abgetrennt. Zur weiteren Reinigung wird das Rohprodukt über eine 10 cm-Vigreux-Kolonne unter Hochvakuum destilliert. Nach Destillation erhält man 306,4 g 2-Picolinsäure-N-tert.-butylamid mit einem Gehalt von 97,6 %.

Ausbeute: 84 % der theoretischen Ausbeute

Beispiel 24.

493,8 g 89,3 %ige Schwefelsäure (6,0 Mol) werden bei Raumtemperatur vorgelegt. Unter Eiskühlung wurden 312,3 g (3,0 Mol) 2-Cyanopyridin so zudosiert, daß eine Innentemperatur von 40°C erreicht wird. Unter Rühren leitet man bei dieser Temperatur innerhalb von 6 h 168,0 g (3,0 Mol) Isobuten ein. Nach Reaktionsende wird auf Raumtemperatur abgekühlt und das Reaktionsgemisch mit 950 ml Wasser verdünnt. Anschließend wird mit 1110 g 45 %iger Natronlauge auf pH 7 gestellt. Das Produkt, das sich als Öl oben abscheidet, wird bei 40°C abgetrennt. Zur weiteren Reinigung wird das Rohprodukt über eine 10 cm-Vigreux-Kolonne unter Vakuum destilliert. Nach Destillation erhält man 326,7 g 2-Picolinsäure-N-tert.-butylamid mit einem Gehalt von 90,0 %.

Ausbeute: 55,0 % der theoretischen Ausbeute

Beispiele 25-34

In Anlehnung an die vorangegangenen Beispiele wurden die Reaktionstemperaturen, die $H_2SO_4$-Mengen und die $H_2SO_4$-Konzentrationen am System 1,0 Mol 2-Cyanopyridin/1,1 Mol tert.-Butanol variiert. In Tabelle 3 sind alle Daten zusammengestellt. Es ist weiterhin angegeben, welche Reaktionskomponente zudosiert wurde (tert.-Butanol oder $H_2SO_4$); die jeweils anderen Reaktionskomponenten (2-Cyanopyridin und $H_2SO_4$ bzw. tert.-Butanol) waren vorgelegt worden.

Tabelle 3 Umsetzung von 1 Mol 2-Cyanopyridin mit 1,1 Mol tert.-Butanol in Gegenwart von Schwefelsäure zu Pyridin-2-carbonsäure-N-tert.-butylamid

| Bsp. Nr. | Reaktions-zeit (h) | Schwefelsäure Konzentr. (%) | Schwefelsäure Menge (Mol) | Temp. (°C) | zudosiert wurde | Ausbeute (%) | restliches 2-Cyano-pyridin (%) |
|---|---|---|---|---|---|---|---|
| 25 | 5 | 90 | 1,1 | 100 | $(CH_3)_3C-OH$ | 88,3 | 6,5 |
| 26 | 2 | 90 | 1,3 | 100 | " | 91,6 | 0,1 |
| 27 | 5 | 90 | 1,3 | 90 | " | 96,5 | 0,2 |
| 28 | 3 | 90 | 1,3 | 80 | " | 95,3 | 1,6 |
| 29 | 7 | 90 | 1,1 | 50 | $H_2SO_4$ | 59,1 | 37,7 |
| 30 | 11 | 90 | 0,8 | 100 | $(CH_3)_3C-OH$ | 79,1 | 15,3 |
| 31 | 2 | 90 | 0,5 | 100 | $H_2SO_4$ | 29,6 | 59,3 |
| 32 | 48 | 90 | 1,5 | 20 | $(CH_3)_3C-OH$ | 60,0 | 30,0 |
| 33 | 48 | 90 | 1,5 | 30 | " | 70,1 | 19,9 |
| 34 | 6 | 90 | 1,8 | 20 | " | 96,0 | 0,2 |

## Patentansprüche

1. Verfahren zur Herstellung von Pyridin-carbonsäure-N-tert.-alkylamiden der Formel

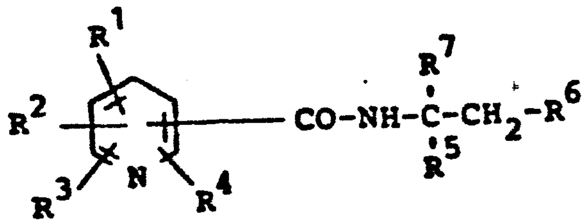

,

in der

$R^1$, $R^2$, $R^3$ und $R^4$ unabhängig voneinander Wasserstoff, Halogen, Alkyl, Alkoxy, Phenyl, Benzyl oder Nitro bedeuten und zwei der Reste $R^1$ bis $R^4$ gemeinsam einen annellierten Benzolring bedeuten können und

$R^5$, $R^6$ und $R^7$ unabhängig voneinander geradkettige oder verzweigte Alkylgruppen mit 1-8 C-Atomen bedeuten, wobei weiterhin $R^5$ und $R^6$ gemeinsam mit den C-Atomen, die sie substituieren, einen aliphatischen Ring von 5-8 Ringgliedern bilden können, weiterhin $R^7$ ein gegebenenfalls substituierter Phenylrest sein kann und $R^6$ außerdem Wasserstoff sein kann,

durch Umsetzung eines Pyridincarbonsäurenitrils der Formel

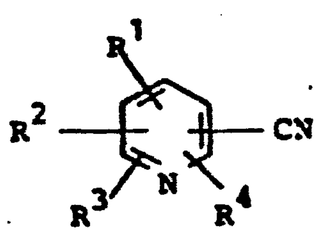

,

in der

$R^1$ bis $R^4$ die angegebene Bedeutung haben,

mit einem tertiäre Carbeniumionen liefernden Alkylierungsmittel der Formel

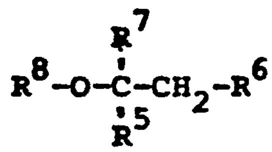

oder der Formel

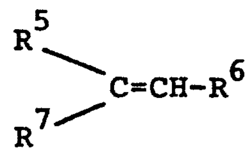

in denen

$R^5$, $R^6$ und $R^7$ die angegebene Bedeutung besitzen und

$R^8$ Wasserstoff, Alkyl oder Acyl bedeuten kann,

in Gegenwart von Schwefelsäure, dadurch gekennzeichnet, daß die Reaktion bei 0 - 100°C und einer Konzentration der Schwefelsäure von 50 - 94 Gew.-% durchgeführt wird.

2. Verfahren nach Anspruch 1, dadurch gekennzeichnet, daß man ein Nitril der Formel

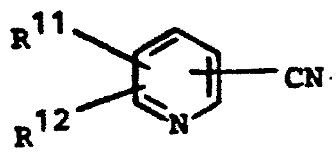

einsetzt,

in der

$R^{11}$ und $R^{12}$ unabhängig voneinander Wasserstoff, Halogen, Alkyl, Alkoxy oder Nitro bedeuten.

3. Verfahren nach Anspruch 1 und 2, dadurch gekennzeichnet, daß man ein tertiäre Carbeniumionen lieferndes Alkylierungsmittel der Formeln

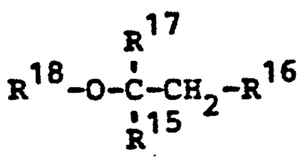

oder

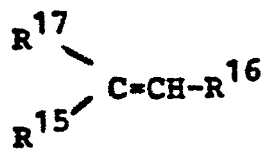

einsetzt,

in denen

$R^{15}$ und $R^{17}$ unabhängig voneinander geradkettiges $C_1$-$C_4$-Alkyl bedueten,

$R^{16}$ für Wasserstoff oder geradkettiges oder verzweigtes $C_1$-$C_4$-Alkyl steht und

$R^{18}$ Wasserstoff, $C_1$-$C_4$-Alkyl oder Acetyl bedeutet,

wobei weiterhin $R^{15}$ und $R^{16}$ gemeinsam mit den C-Atomen, die sie substituierten, einen aliphatischen Ring mit 5-6 Ringgliedern bilden können.

4. Verfahren nach Anspruch 1 bis 3, dadurch gekennzeichnet, daß 60-90 %ige Schwefelsäure eingesetzt wird.

5. Verfahren nach Anspruch 1 bis 4, dadurch gekennzeichnet, daß 0,5-3,0 Mol Schwefelsäure pro Mol Nitril eingesetzt werden.

6. Verfahren nach Anspruch 1 bis 5, dadurch gekennzeichnet, daß 0,8-2,7 Mol Schwefelsäure, besonders 1,0-2,5 Mol Schwefelsäure, pro Mol Nitril eingesetzt werden.

7. Verfahren nach Anspruch 1 bis 6, dadurch gekennzeichnet, daß 0,8-4,0 Mol, bevorzugt 0,8-2,5 Mol, besonders bevorzugt 1,0-2,0 Mol Alkylierungsmittel pro Mol Nitril eingesetzt werden.

8. Verfahren nach Anspruch 1 bis 7, dadurch gekennzeichnet, daß ohne weiteres Lösungsmittel gearbeitet wird.

9. Verfahren nach Anspruch 1 bis 8, dadurch gekennzeichnet, daß ein Gemisch aus Nitril und Schwefelsäure vorgelegt wird und das Alkylierungsmittel zudosiert wird.

10. Verfahren nach Anspruch 9, dadurch gekennzeichnet, daß bei einer Temperatur von 20 - 90°C, bevorzugt bei einer Temperatur von 50 - 90°C gearbeitet wird.